# EUROPEAN PATENT APPLICATION

(11) **EP 1 849 861 A1**
(43) Date of publication of application: **31.10.2007**
(21) Application number: 06714013.7
(22) Date of filing: 17.02.2006
(51) Int. Cl.: C12N 5/00, C12M 1/00, C12M 1/42, C12N 13/00, C12Q 1/02

(54) **METHOD AND APPARATUS FOR SEPARATING CELLS**

(30) Priority: 18.02.2005 JP 2005042378
(71) Applicant: ABsize Inc., Osaka-shi, Osaka 530-0053 (JP)
(72) Inventor: NIWA, Hideo, Akashi-shi, Hyogo 6740084 (JP); KOBAYASHI, Akira, Tatsuki-shi, Osaka 5690826 (JP); SATO, Setsuya, Otokuni-gun, Kyoto 6180071 (JP); MATSUMOTO, Yoshitaka, Kyoto-shi, Kyoto 6128089 (JP); OH, Isamu, Sennan-gun, Osaka, 5980093 (JP)
(74) Representative: Copp, David Christopher
(86) International application number: PCT/JP2006/302872
(87) International publication number: WO 2006/088154

(57) **Abstract**

The present invention provides a method and an apparatus for selectively removing non-target cells from a mixed cell population of different types of cells to selectively separate target cells. Individual cells or cell groups of at least two cells from a mixed cell population of different types of cells are placed on a certain two-dimensional coordinate system. Then a distinction is made among the individual cells or cells in the cell groups, based on the shape or size of the cells or by using a cell marker. Physical energy such as laser light is applied to the positions or regions occupied by non-target cells or cell groups including non-target cells in order to selectively kill the non-target cells or cause dysfunction of the non-target cells.

## Description

### Field of the Invention

The present invention relates to a method and an apparatus for separating target cells. According to the present invention, individual cells or cell groups of at least two cells are located on a substrate. Then non-target cells or cell groups including non-target cells are distinguished from target cells or cell groups including target cells based on the size or shape of the cells or by using a cell marker. Laser light is applied to the non-target cells or the cell groups including non-target cells so as to selectively kill the non-target cells or cause dysfunction of the non-target cells.

### Background Art

Selective separation of particular cells from a mixed cell population of various types of cells is an important technology in analyses of the functions and genes of cells and in the fields of analysis, diagnosis, and treatment using cells. Particularly in the field of recent regenerative medicine and cell therapy using cells, there is a high expectation for the development of a technology of preparing safe and therapeutic cells to minimize contamination caused by non-targeted cells.

Conventionally, cell sorters have been widely used as apparatuses to selectively separate cells. Cell sorters are characterized in that they enable a rapid simultaneous treatment of a large quantity of specimens, but they have some problems. For example, cells must be subjected to chemical or biochemical treatment or treatment under an undesirable condition for the cells. A cell separation method using magnetic beads coupled with an antibody that selectively binds to a certain type of cells has also been devised. This method shows considerably low cell recovery rate and therefore is not desirable for cell separation used for analysis, diagnosis, and treatment. A system for applying laser light to certain cells in a cell population so as to kill non-target cells and selectively obtain target cells has also been devised (refer to Patent Document 1 and Non-patent Document 1). The separation efficiency of this system is not sufficient because a process of identifying the target cells to be irradiated with laser light among a cell population and a process of laser irradiation must be performed in accordance with the number of cells. One of other problems of this system is that laser light might not sufficiently irradiate cells due to the differences in size and shape of cells, and resultantly the efficiency of separation is decreased.
Patent Document 1: WO01/40454, Method and Apparatus for Selectively Targeting Specific Cells within a Cell Population
Non-patent Document 1: Niemz M. H., Laser-tissue interaction: Fundamentals and applications. Springer-Verlag, 1996

### Disclosure of the Invention

### Problems to be solved by the invention

The present invention provides a method and an apparatus for separating target cells by identifying target cells and non-target cells among a mixed cell population of different types of cells based on the shape or size of individual cells or of cell groups in the cell population or by using a cell-specific label, and selectively applying physical energy to the non-target cells to kill the non-target cells or cause dysfunction of the non-target cells.

### Means to-Solve the Problems

It is possible to achieve highly efficient separation of target cells by positioning individual cells or cell groups of at least two cells in a mixed cell population including target cells and non-target cells to particular positions or areas using a substrate patterned with cell-adhesive areas, and selectively applying laser light to the positions or areas occupied by non-target cells or cell groups including non-target cells to kill the non-target cells or induce dysfunction of the non-target cells.

The present invention provides a cell separation method comprising: placing individual cells or cell groups consisting of at least two cells in certain positions on a substrate; distinguishing target cells or cell groups including target cells from non-target cells or cell groups including non-target cells, based on shape or size of the individual cells or of cells in the cell groups, or by using a cell marker; and applying laser light to the positions or regions in which the non-target cells or the cell groups including non-target cells are placed in order to selectively kill the non-target cells or cause dysfunction of the non-target cells.

The present invention also provides a cell separation method comprising: placing individual cells or cell groups consisting of at least two cells in certain positions on a substrate; distinguishing target cells or cell groups including target cells from non-target cells or cell groups including non-target cells, based on shape or size of the individual cells or of cells in the cell groups, or by using a cell marker; applying laser light to the positions or regions in which the non-target cells or the cell groups including non-target cells are placed in order to selectively kill the non-target cells or cause dysfunction of the non-target cells; and selectively culturing only the target cells.

Further, the present invention provides a cell separation apparatus comprising: a mechanism configured to place individual cells or cell groups consisting of at least two cells in certain positions on a substrate; a mechanism configured to distinguish target cells or cell groups including target cells from non-target cells or cell groups including non-target cells, based on shape or size of the individual cells or cells in the cell groups, or by using a cell marker; and a mechanism configured to apply laser light to the positions or regions in which the non-target cells or the cell groups including non-target cells are placed.

Hereinafter, the method and apparatus of the present invention for efficiently separating target cells alone by killing non-target cells or causing dysfunction of non-target cells with laser light to remove the non-target cells from a cell population consisting of target cells and non-target cells according to the present invention will be described.

According to the cell separation method of the present invention, first, individual cells or cell groups in a mixed cell population of target cells and non-target cells are arranged in certain positions on a substrate. Next, non-target cells or cell groups including non-target cells are identified among the arranged cells based on their shape or size or by using a cell marker. Then laser light is selectively applied to the identified positions or areas to selectively kill non-target cells or cause dysfunction of non-target cells and thereby selectively separate target cells.

If necessary, a cell population in which non-target cells were killed or dysfunction is caused in non-target cells may be cultured under a condition suitable for target cells in order to obtain target cells. Such method of obtaining target cells is also included in the present invention.

The target cells may be, but not limited to, chondrocytic cells differentiation-induced from a cluster of cells including mesenchymal stem cells by TGF-β (transforming growth factor β) or the like.

The non-target cells may be, but not limited to, cells not differentiated into target cells (that is, chondrocytic cells) or cells differentiated to cells other than the target cells, in the above-mentioned differentiation-inducing system from a cluster of cells containing the mesenchymal stem cells to chondrocytic cells.

First, according to one method used for arranging individual cells or cell groups on a substrate, individual cells or cell groups are placed on cell-adhesive surfaces positioned in a pattern on a substrate with a non-cell-adhesive surface.

This method of locating individual cells or cell groups on certain positions in a substrate is preferable because the method makes it easy to recognize the location of non-target cells as well as the method makes it possible to kill non-target cells or cause dysfunction of non-target cells by laser light in a reliable and safe way.

To obtain cell-adhesive surfaces placed in a pattern on a non-cell-adhesive surface, for example, cell-adhesive surfaces of cell-adhesive agent may be placed in a pattern on a substrate with a non-cell-adhesive surface. Alternatively, non-cell-adhesive surfaces of non-cell-adhesive agent may be formed on a substrate with a cell-adhesive surface so as to expose cell-adhesive surfaces in a pattern.

The substrate with a non-cell-adhesive surface may be any substrate that does not adhere or bind to cells, but the material of such substrate is preferably glass, silicon compounds, or non-cell-adhesive polymers (for example, resins such as polystyrene). Optionally, the surface of the substrate may be surface-coated or surface-modified with hydrophilic polymers. Such hydrophilic polymers may include, but not limited to, polyvinyl alcohol, polyethylene glycol, polyacrylamide, polydimethylacrylamide, and polyhydroxyethylemethacrylate; as well as copolymers of the monomers constituting these polymers; and cellulose. In addition, the non-cell-adhesive agent may be, for example, the same material as the substrate with a non-cell-adhesive surface.

The cell-adhesive agent and the material of the substrate with a cell-adhesive surface are not particularly limited but may be any agent or material that adheres or binds to cells. The agent or material may include metal-oxides, cell-adhesive proteins and their derivatives, temperature-sensitive polymers, light-curing resins, and other cell-adhesive polymers (for example, polysaccharide), as mentioned later.

Methods to form a cell-adhesive surface may include, but not limited to, a method of immobilizing cell-adhesive polymers as disclosed in Japanese Patent Publication H7-308186, and a method of forming a pattern of cell-adhesive polymers on the surface of a substrate by ink jet process as disclosed in Japanese Patent Publication 2002-355026. Where the substrate surface includes glass or a reactive functional group such as hydroxyl, amino, or thiol, it is also possible to use a method of adding alcohol, alkyl halide, organic silane compounds to the substrate surface by stamping or the like to add a hydrophobic substituent group.

To form a cell-adhesive surface on a substrate with a non-cell-adhesive surface, surface modification using metal oxide formed by physical deposition methods such as vacuum evaporation or sputtering, chemical vapor deposition methods, electrochemical coating methods such as plating, or the like may be used. The metal oxide is preferably, but not limited to, titanic oxide. In addition, it is also possible to use the cell-adhesive surface absorbed or immobilized with cell-adhesive proteins such as gelatin, collagen, fibronectin, and laminin or the derivatives of the segments of these proteins.

In addition, as a method to form a cell-adhesive surface on a substrate with a non-cell-adhesive surface, for example, a pattern-forming method using a temperature-sensitive polymer such as poly(N-isopropyl acrylamide) as disclosed in Japanese Patent Publication H4-094679 may be used. Particularly, the cell-adhesive surface formed by the polymer becomes a non-cell-adhesive surface at lower temperatures than the temperature at which a decrease occurs in the hydrophobic property of the polymer layer coated on the surface.

The present invention includes a method to recover target cells which have not been killed or whose functions were not damaged in order to culture those target cells in a suitable condition, in addition to killing non-target cells or causing dysfunction of non-target cells by laser light. In this regard, it is preferable to form the cell-adhesive surface with temperature-sensitive polymers because such cell-adhesive surface eliminates the need for using trypsin or the like in recovering target cells from the substrate.

Methods to form non-cell-adhesive surfaces on a substrate with cell-adhesive surface to expose a pattern of cell-adhesive surfaces on the substrate with a cell-adhesive surface include a method of immobilizing non-cell-adhesive polymers, disclosed in Japanese Patent Publication H7-308186, a method of immobilizing a non-cell-adhesive surface of silicon compounds to a cell-adhesive substrate, disclosed in Japanese Patent Publication H11-151086, and a method of forming microwells with bottom surfaces formed from a substrate with cell-adhesive surfaces of light-curing resin.

The light-curing resin used for producing the microwells may be any resin which, once cured, exhibits low cell-adhesiveness and sufficient cell compatibility, but may include, for example, a light-curing resin that contains a compound with an oxetane ring, a compound with an epoxy group, cation-series light-polymerization initiator, which is disclosed in Japanese Patent Publication H10-168165.

The size and shape of the cell-adhesive surface formed on a substrate with a non-cell-adhesive surface and those of the patterned cell-adhesive surfaces exposed on a substrate with a cell-adhesive surface are not particularly limited. The preferable size of the cell-adhesive surface is 20µm²-400 µm² for arranging individual cells, or 200µm²-1000 µm² for arranging cell groups of at least two cells. The shape of the cell-adhesive surface may be, for example, circular and rectangular.

The substrate used for arranging the cells employed in the present invention may be, for example, a Petri dish, a culturing flask, a sealed cell culturing apparatus. To maintain the viability of cells and prevent external contamination, sealed cell culturing apparatus is preferable. The sealed cell culturing apparatus may have a structure or a device for circulating culturing liquids.

Next, a method of separating target cells by identifying non-target cells or cell groups including non-target cells among individual cells or cell groups located on a substrate, and applying laser light selectively to the positions or areas where the non-target cells or cell groups including non-target cells are arranged will be described.

Non-target cells or cell groups including non-target cells are identified among the individual cells or cell groups arranged on the substrate, based on the size or shape of the cells or by using a cell marker. Then the positions occupied by the non-target cells or the cell groups including non-target cells are identified.

The distinction based on the size or shape of the cells or by using a cell marker is performed by microscopic observation, or fluorescent microscopic observation using fluorescently labeled cells.

In the distinction of cells based on their size or shape, the shape of the cells is identified as globular, spindle-like, stone-flagged, or dendritic based on microscopic images or fluorescent microscopic images of fluorescently labeled cells. The size of the cells is identified based on the major or minor axis of the cells or the combination thereof, using microscopic images, or fluorescent microscopic images of fluorescently labeled cells.

In the distinction of cells using a cell marker, antibodies which selectively bind to the marker molecules in the non-target cells; direct or indirect fluorescent labeling using peptides, lectin, sugar chains, or the like; or pigments which selectively bind to the membranes of non-target cells may be used.

It is preferable to use the distinction by size or shape of the cells because the distinction by cell markers requires a process of allowing the marker molecules to recognize the cells. When the cells are distinguished based on their size or shape, it is possible to perform a more accurate quantitative determination using image analysis software or the like.

Observed images of individual cells or cell groups are captured by a CCD camera, and then transferred to an image processor such as a personal computer. By using the available positions for cell placement in the substrate as reference positions, it is recognized whether the individual cells or cell groups that are present at the reference positions are the non-target cells or cell groups including non-target cells. This information is transferred directly to a laser irradiation device or to a storage device. In the image capturing by a CCD camera, image capturing and image data transfer to an image processor are repeated depending on the recognizable range of a field of view of a CCD element to be used and on the size of the substrate on which the cells are located, until the whole area of the substrate on which the cells are located is scanned. The repeating process is performed by moving the stage supporting the substrate in the X and Y directions, by moving the image processor that includes a CCD camera in the X and Y directions, or by using a combination thereof.

In the present invention, halogen lamps, LEDs, and LDs may be used as a light source for image observation using transmissive light, whereas halogen lamps, LEDs, and LDs may be used as a light source for observing fluorescent images. In obtaining fluorescent images, absorption filters or spectrometers are used to remove the diffused exiting beams with a wavelength other than that of the fluorescent light for observation.

Then, based on the image processing information of the cells on the substrate obtained in this way, laser light is selectively applied to the non-target cells or cell groups including non-target cells to kill the non-target cells or cause dysfunction of the non-target cells.

In the laser irradiation, either spot irradiation to the areas occupied by non-target cells or cell groups including non-target cells, or irradiation of patterned laser light in accordance with the pattern of the areas occupied by non-target cells or cell groups including non-target cells may be used.

The laser light is transferred, via a lens system, to an image processor such as a personal computer or optionally to a storage device, and applied to non-target cells or the positions occupied by non-target cells.

In the spot irradiation of laser light, the spot size is adjusted depending on the cells on the adhesive surface of the cells or cell groups on the substrate to be used, using a combination of the lens system, so that laser light is applied to the whole adhesive surface of the cells or cell groups.

Laser irradiation to all of the non-target cells or the cell groups including non-target cells which are present on the substrate is enabled by moving the XY stage supporting the substrate on which those cells are located, laser beam scanning using a powered mirror such as a galvano-mirror, or using a combination thereof. In the laser beam scanning using a powered mirror, more efficient laser irradiation is enabled by use of a powered mirror (galvano-mirror), as disclosed in Japanese Patent Publication H4-334544. Combination of a powered mirror (e.g., the galvano-mirror) with a scanning lens (e.g., the fθ lens) is preferably used because it enables a broader laser scan as well as it eliminates or minimizes the need to move the stage supporting the substrate.

Irradiation of patterned laser light facilitates simultaneous irradiation to multiple non-target cells or of areas occupied by non-target cells and thereby improves the efficiency of irradiation to non-target cells or cell groups including non-target cells. Also in the patterned laser irradiation, a galvano-mirror (or combination of a galvano-mirror and an fθ lens) is preferably used to treat cells over a larger area without moving the XY stage supporting the substrate.

For patterning laser according to the present invention, an element (e.g., Digital Mirror Device: DMD) that includes a series of mirrors, each capable of independent angle adjustment, and a spatial light modulation element which alters the phases of light to control light thickness, a liquid crystal filter, or the like, may be used.

In the present invention, the intensity of the laser applied to non-target cells or cell groups including non-target cells is adjusted to an extent that the laser has sufficient energy intensity to kill cells or cause dysfunction of cells and that the laser does not influence the adjacent target cells or target-cell groups.

The laser source used in the present invention may be, but not limited to, excimer laser, solid laser, or semiconductor laser with adequate laser intensity. Preferably, the laser source is pulsed laser because it is expected that pulsed laser irradiation induces multiphoton absorption resulting from the nonlinear optical effect so as to cause local optical reaction in cells and increase the effect of killing cells or causing dysfunction of cells. In addition, high repetitive laser source is preferable to improve the processing efficiency used.

To achieve such a multiphoton absorption process, it is known that pulse width of the laser light to be applied must be shorter than the time scale in which optical energy is converted into molecular heat energy. Since the time scale is considered to be several tens of nanoseconds to several hundreds of picoseconds, the upper limit of the pulse width of the laser light is preferably 10 ns or less, more preferably 5 ns or less, and most preferably 1 ns or less. The lower limit is preferably 50 fs or more, and more preferably 100 fs or more.

While it is preferable to use a pulsed laser source with a high repetitive frequency in order to improve processing efficiency, the repetitive frequency between 20 kHz and 50 kHz is desirable because the peak power for each laser pulse is decreased at a higher repetitive frequency

Considering these conditions, it is desirable that the laser light has an output of 1-20 W, a pulse width of 100 fs to 10 ns, and a repetitive frequency of 20-50 kHz.

The energy applied to cells or cell groups is adjusted with laser irradiation time and laser output. It is more preferable to use a shutter to control the irradiation time of laser light.

Acoustooptical modulation elements (AOM) are capable of high-speed processing with a maximum frequency of around 35-50 MHz, which is faster than the repetitive frequency of laser light. Thus, the processing speed of killing cells or causing dysfunction of cells depends on the repetitive frequency of the laser source.

In addition, the wavelengths of the laser light to be used may be preferably 300 to 1100 nm, but not limited to this range. In case of using laser light with a wavelength of 400 nm or above, compounds such as pigments absorbing the light with such wavelength is added to cell culture solution to more effectively kill cells or cause dysfunction of cells. As a non-limiting example, allura red may be added where laser light with a wavelength of 532 nm is used.

In addition, the intensity of the laser light to be applied may be adjusted by optical elements. For example, an ND filter, a combination of a plate with 1/2λ wavelength and a polarized beam splitter, or an acoustooptical modulation elements (AOM) may be used.

In case of using an ND filter, an ND filter, which is formed from materials absorbing or reflecting a certain quantity of light and which do not influence other components of the laser light than light intensity is placed on the light axis of the laser light for allowing attenuation of the laser light.

In case of using a combination of a 1/2λ wavelength plate and a polarized beam splitter, 1/2λ wavelength plate is used for changing the polarization direction of the linearly- polarized laser light, and a beam splitter is used for changing the ratio of separation between the transmitted beams and reflected beams, so as to alter laser light intensity.

In case of using acoustooptical modulation elements (AOM) generates a compressional wave of the refractive index in the elements depending on the modulation signal provided to the elements (ultrasonic wave). The compressional wave is used as a diffractive grating to vary the intensity of the diffractive light caused by modulating the intensity of the ultrasonic wave. By allowing the laser light to transmit through the elements and recovering the diffracted beams, laser light with a regulated optical intensity can be obtained.

In this way, it is possible to selectively kill non-target cells or cause dysfunction of non-target cells to separate target cells.

If necessary, the cell population in which non-target cells have been killed or dysfunction is caused in non-target cells may be cultured under a suitable condition for the target cells in order to obtain target cells.

Next, the cell separation apparatus of the present invention is described.

The cell separation apparatus of the present invention includes a mechanism to locate individual cells or cell groups of at least two cells on a substrate, a mechanism to distinguish non-target cells or cell groups including non-target cells from target cells or cell groups including target cells based on the size or shape of the cells or by using a cell marker, and a mechanism to apply laser light to the positions or areas occupied by non-target cells or cell groups.

The cell separation apparatus may include, if necessary, a mechanism to integrally control the above-mentioned mechanisms.

To apply laser light to the positions occupied by the identified non-target cells or cell groups including non-target cells, according to the arrangement pattern of those cells or cell groups, the cell separation apparatus preferably includes a mechanism for patterning laser light according to the arrangement pattern and applying laser light simultaneously to multiple non-target cells or cell groups including non-target cells.

As the mechanism to apply laser light to the positions occupied by the recognized non-target cells or cell groups including non-target cells according to the arrangement pattern of those cells or cell groups, the cell separation apparatus preferably includes a mechanism consisting of an element for forming a pattern of laser light and an element for deflecting these patterned laser light, and a mechanism consisting of a scanning lens for focusing the deflected laser light on the positions occupied by the cells or the cell groups.

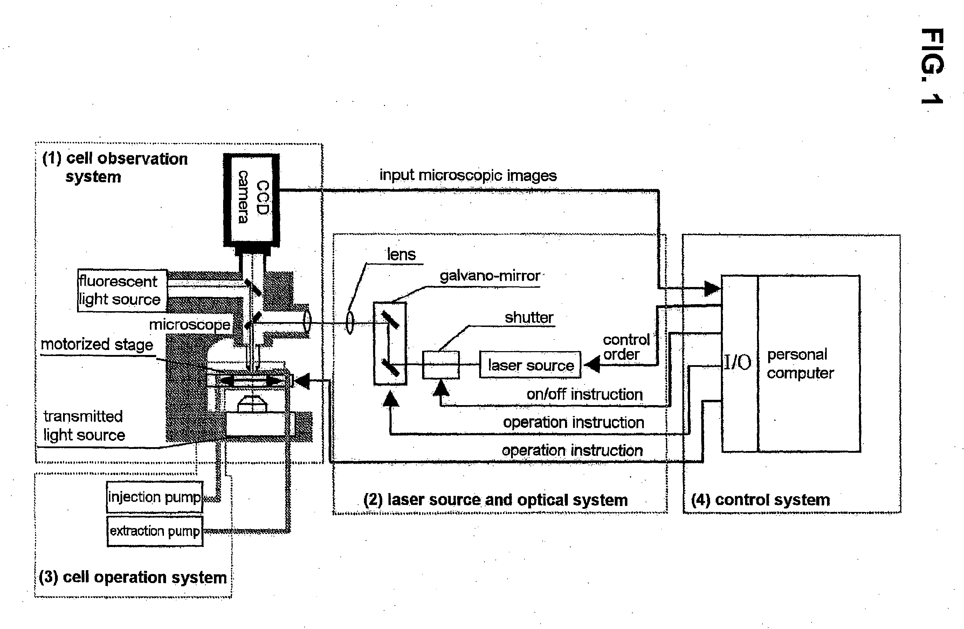

Fig. 1 shows one embodiment of the cell separation apparatus according to the present invention.

The cell separation apparatus includes four systems: (1) a cell observation system; (2) a laser source and optical system; (3) a cell operation system; and (4) a control system.

The mechanism to locate cells, the mechanism to distinguish cells and the mechanism to perform irradiation as mentioned above respectively correspond to (3) cell operation system, (1) cell observation system and (2) laser source and optical system.

The cell observation system (1) includes a microscope to observe cells, and the microscope includes a CCD camera to capture microscope images. The microscope also includes a transmitted light source as well as a fluorescent light source to observe fluorescently labeled cells. Images of cells captured by a CCD camera are transferred to a personal computer, where non-target cells are distinguished from target cells so as to determine the positions or areas occupied by the non-target cells. The cell observation system includes an electrically controlled substrate stage to allow the cells to be observed over the whole area of the substrate.

The laser source and optical system (2) includes a laser source with an output sufficient for killing cells or causing dysfunction of cells through laser irradiation. A shutter is installed on the optical axis to apply laser light when it is required. The laser light is introduced into the microscope via a galvano-mirror, which is a motorized mirror, to apply focused laser light to any recognized position in the microscopic field. This enables XY scanning of the irradiation positions of the focused laser light. On the other hand, the focal positions and the spot size of the focused laser light at the focal positions in the field of view of the microscope are controlled by a lens external to the microscope.

In the cell operation system (3), cells are seeded in a culture vessel having a pattern of cell-adhesive surfaces provided on a non-cell-adhesive surface, and the cells are arranged on the pattern. A motorized stage to move the culture vessel in the X and Y directions is used to apply laser to the whole area of the culture vessel containing the cells arranged on the pattern. The culture vessel containing the arranged cells is connected to a pump to supply, recover, or circulate cell seeding solutions, culture fluids, cell recovery fluids, or the like.

The control system (4) is configured to acquire images of the cells being observed and to recognize the presence of target cells and non-target cells for each patterned area. The angle of the galvano-mirror and the position of the lens external to the microscope are controlled so that laser light is applied to the area occupied by the non-target cells. The control system is also configured to control the shutter and the intensity of the light source.

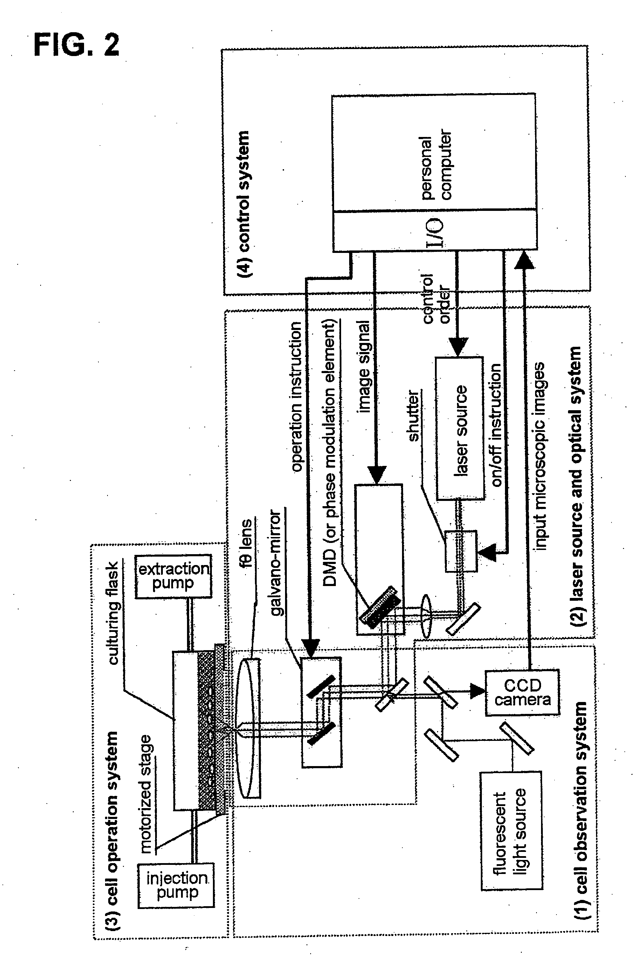

Fig. 2 shows another embodiment of the cell separation apparatus according to the present invention.

The cell separation apparatus consists of four systems: (1) a cell observation system; (2) a laser source and optical system; (3) a cell operation system; and (4) a control system.

The cell observation system (1) has a CCD camera used for observing cells and a fluorescent light source used for recognizing the cells, so that the appearance of the cells to be observed in the culture vessel may be recognized via an fθ lens (i.e., scanning lens) and a galvano-mirror. Scanning the galvano-mirror allows observation of a large area without moving the stage.

The laser source and optical system (2) use a combination of lens systems including one lens for magnifying laser beams from the laser source for ensuring even intensity distribution, and the other lens for changing the laser beams into parallel beams. A DMD element or a phase modulation element is used to alter the beam pattern of the magnified uniform beams according to the recognized patterned area. These patterned beams are passed through the galvano-mirror and fθ lens to achieve simultaneous irradiation of the laser beams to all of the identified areas among the patterned areas under observation. Passing laser beams deflected by a galvano-mirror through the fθ lens enables the laser beams to be focused on the plane on which the cells are present. A shutter is installed on the light axis to apply laser light when it is required.

In the cell operation system (3), cells are seeded in a culture vessel having a pattern of cell-adhesive surfaces provided on a non-cell-adhesive surface, and the cells are arranged on the pattern. A motorized stage to move the culture vessel in the X and Y directions is used to apply laser to the whole area of the culture vessel containing the cells arranged on the pattern. The culture vessel containing the arranged cells is connected to a pump to supply, recover, or circulate cell seeding solutions, culture fluids, cell recovery fluids, or the like. In such a cell operation system, cultivation, observation, and laser processing are performed simultaneously.

The control system (4) is configured to acquire images of the cells being observed and to recognize the presence of target cells and non-target cells for each patterned area, as described later in Example 1. By analyzing the results of recognition, the beam pattern to be emitted is calculated so as to apply laser light simultaneously to all the areas where non-target cells are recognized. These calculation results are transferred to the element for patterning beams so as to allow beams with a given pattern to be applied. To view all observation areas, the areas which can be recognized in one field of view are sequentially scanned by using the galvano-mirror. This facilitates observation and laser irradiation without influencing the status of the cells.

### Effect of the Invention

The methods and apparatuses of the present invention allow highly efficient separation of target cells without the need to chemically or physically treat them by selectively removing non-target cells from a mixed cell population of different types of cells. The cells or cell groups separated by the present method and apparatus are suitable for the analyses of the functions and genes of cells, analyses of the functions of agents using cells, and the application to medical fields utilizing cells.

### Description of the Preferred Embodiments

Hereinafter, the present invention is described in detail referring to an example shown below. The present invention, however, is not limited to this example.

### Example 1

Mesenchymal cells were separated from bone-marrow cells as follows:
1) Bone-marrow fluid taken from the humeral head of a Japanese white rabbit by bone marrow tapping was mixed with the same volume of physiologic saline (supplied by Otsuka Pharmaceutical Co., Ltd.) containing heparin sodium (supplied by Shimizu Pharmaceutical Co., Ltd.,) at 1 u/mL. Then the mixture was centrifuged at 1200 rpm for ten minutes to separate blood cells.
2) The separated blood cells were added into a T-75 flask (IWAKI 3110-075) containing α MEM (Invitrogen 12571-063) supplemented with 15% fetal bovine serum (Invitrogen 10099-141) and antibiotic-antifungal agents (Invitrogen 15240-062). After the blood cells were cultured for two days, adhesive cells alone were separated.
3) The present adhesive cells were dispersed into the α MEM. Then, these cells were added to a substrate formed with a glass base provided with microwells of light-curing resin (D-MEC SCR950) with a diameter of 100 µm, and cultured for 12 hours.
4) After non-adhesive cells outside the microwells were washed off, anti-CD34 antibody labeled by phycoerythrin (PE), which is a fluorescent dye, was added to the microwells. Then, the microwells containing CD34-positive cells were identified by fluorescent observation, and 355-nm laser light was applied to these microwells. The intensity of the laser was 155 W/cm², and the laser light was irradiated to whole areas of the microwells for 10 seconds. Trypan blue staining test on the viability of cells was performed after the laser irradiation and the result showed that cells were killed in all the irradiated areas of microwells.
5) After the laser irradiation, the substrate was washed with PBS buffer (phosphoric acid buffered physiologic saline) to remove debris derived from the killed cells. Then the unirradiated cells were recovered through trypsin treatment. The recovered cells were added again into a T-75 flask (IWAKI 3110-075) containing α MEM (Invitrogen 12571-063) to which 15% fetal bovine serum (Invitrogen 10099-141) and antibiotic-antifungal agents (Invitrogen 15240-062). The cells were cultured for two days, to obtain mesenchymal stem cells useful for the regeneration of cartilage.

### Industrial Applicability

The methods and apparatuses of the present invention allow highly efficient separation of target cells without the need to chemically or physically treat them by selectively removing non-target cells from a mixed cell population of different types of cells. The cells or cell groups separated by the present method and apparatus are suitable for the analyses of the functions and genes of cells, analyses of the functions of agents using cells, and the application to medical fields utilizing cells.

### Brief Description of the Drawings

Fig. 1 shows one example of the configuration of the cell separation apparatus according to the present invention; and
Fig. 2 shows one example of the configuration of the cell separation apparatus according to the present invention.

## Claims

1. A cell separation method comprising:
placing individual cells or cell groups consisting of at least two cells in certain positions on a substrate;
distinguishing non-target cells or cell groups including non-target cells from target cells or cell groups including target cells, based on shape or size of the individual cells or of cells in the cell groups, or by using a cell marker; and
applying laser light to the positions or regions in which the non-target cells or the cell groups including non-target cells are placed in order to selectively kill the non-target cells or cause dysfunction of the non-target cells.

2. A cell separation method comprising :
placing individual cells or cell groups consisting of at least two cells in certain positions on a substrate;
distinguishing non-target cells or cell groups including non-target cells from target cells or cell groups including target cells, based on shape or size of the individual cells or of cells in the cell groups, or by using a cell marker;
applying laser light to the positions or regions in which the non-target cells or the cell groups including non-target cells are placed in order to selectively kill the non-target cells or cause dysfunction of the non-target cells; and
selectively culturing only the target cells.

3. A cell separation apparatus comprising:
a mechanism configured to place individual cells or cell groups consisting of at least two cells in certain positions on a substrate;
a mechanism configured to distinguish non-target cells or cell groups including non-target cells from target cells or cell groups including target cells, based on shape or size of the individual cells or cells in the cell groups, or by using a cell marker; and
a mechanism configured to apply laser light to the positions or regions in which the non-target cells or the cell groups including non-target cells are placed.

4. The cell separation method of claim 1 or 2, or the cell separation apparatus of claim 3, wherein the individual cells or the cell groups consisting of at least two cells are placed on cell-adhesive surfaces which are arranged in a pattern on a non-cell-adhesive surface of the substrate.

5. The cell separation method of claim 1 or 2, or the cell separation apparatus of claim 3, wherein the individual cells or the cell groups consisting of at least two cells are placed on microwells having exposed cell-adhesive surfaces.

6. The cell separation apparatus of claim 3, comprising a mechanism configured to apply laser light to positions in which the recognized non-target cells or cell groups including non-target cells according to an arrangement pattern of the non-target cells or cell groups including non-target cells by patterning laser light according to the arrangement pattern and applying laser light simultaneously to a plurality of non-target cells or to a plurality of cell groups including non-target cells.

7. The cell separation apparatus of claim 3 or 6, comprising:
a mechanism including an element configured to form a pattern of the laser light and an element configured to deflect the patterned laser light; and
a mechanism including a scanning lens configured to allow the deflected laser light to be focused on positions in which non-target cells or cell groups including non-target cells,
wherein the mechanisms are used to apply laser light to positions in which the recognized non-target cells or cell groups including non-target cells according to an arrangement pattern of the non-target cells or cell groups including non-target cells.

8. The cell separation method of claim 1 or 2, or the cell separation apparatus of claim 3, wherein the laser light to be applied to cells is pulsed laser.
